# EUROPEAN PATENT APPLICATION

(11) **EP 3 035 050 A1**
(43) Date of publication of application: **22.06.2016**
(21) Application number: 15195841.0
(22) Date of filing: 23.11.2015
(51) Int. Cl.: G01N 33/20, G01N 25/04, G01N 1/12

(54) **A SAMPLING DEVICE FOR THERMAL ANALYSIS**

(30) Priority: 16.12.2014 SE 1451548
(71) Applicant: SinterCast AB, 100 55 Stockholm (SE)
(72) Inventor: ELMQUIST, Lennart, 554 46 Jönköping (SE); POPELAR, Patrik, 641 53 Katrineholm (SE)
(74) Representative: Zacco Sweden AB

(57) **Abstract**

A sampling device for thermal analysis of solidifying metal is disclosed. The sampling device comprises at least a first cavity and a second cavity, the first and second cavities adapted to be filled with a molten metal to be analysed and each cavity adapted to comprise a temperature responsive means during thermal analysis. The inlet opening of the common filling inlet is arranged in a second plane essentially parallel to a first horizontal plane tangent to an uppermost portion of the sampling device and located below said first plane.

## Description

### Field of Invention

The present invention relates to a sampling device for thermal analysis of solidifying metal, especially for thermal analysis in the production of castings.

### Background

Thermal analysis is a technique monitoring variations in temperature change of certain molten substances during solidification and subsequent solid state transformations to be able to determine the microstructure and hence properties of the substances in solid form. This is accomplished by obtaining a representative sample from the melt, transferring it into a sample vessel and recording and evaluating a time-dependent temperature change in the sample during solidification, by means of temperature responsive means, such as thermocouples or other devices known in the art.

When using thermal analysis for controlling solidification processes in molten materials, such as cast-iron or aluminium alloys, a most critical issue is to provide for a controlled, even and reproducible rate of heat removal from the sample. The reason for this is to make it possible to measure temperature changes during phase transformations, the knowledge of which is essential in order to predict microstructures and control certain solidification processes.

Thermal analysis is a heat balance. The ultimate shape, and thus the resolution, of the cooling curve is determined by the balance between the latent heat liberated during solidification and the heat transferred from the metal to the sampling device and from the sampling device to the atmosphere. It is evident that the amount of heat liberated by the solidification of a 200 gram sample of, for example cast iron, is fixed. If the 200 gram sample is contained in a vessel that has a high heat capacity, the heat liberated by the solidification will be less able to prevail over the heat transferred to and from the vessel. The result is that a sampling vessel with high thermal mass and/or a high cooling rate will provide less resolution in the cooling curves. High heat transfer from the vessel to the atmosphere will also result in fast cooling of the sample, which influences the development of the microstructure. For instance, fast cooling caused by the vessel will alter the solidification behaviour of the iron by inducing chill by increasing the undercooling. In order to extract as much information as possible from the heat liberated by the solidification, it is necessary to design a thermal analysis sampling device such that it neither masks nor dilutes the information provided by the solidification. The other major requirement of a thermal analysis sampling device is that it must ensure consistent sampling conditions. Because the differences in the liberated heat between a good microstructure and an out-of-spec microstructure can be very small, it is critical that all variations measured are due to differences in the material to be analysed and not due to differences in the sampling technique.

Sampling devices for thermal analysis may be constructed of a variety of materials as long as the material is able to withstand the temperature of the melt to be analysed at least in the sense of being dimensionally stable at the high temperature and not causing any undesired reaction with the molten sample. For example, it is previously known to construct the sampling devices from graphite, ceramic materials, steel, or a combination thereof.

Thermal analysis sampling devices commonly used in the evaluation of cast iron microstructures are typically constructed of chemically bonded sand, also known as "sand cups" within the technical field. These types of sampling devices do however have certain shortcomings which affect the accuracy of microstructure prediction during thermal analysis. Sand cups typically have thick sand walls to ensure safe containment of the molten iron, which results in high heat capacity causing the vessel to serve as a heat sink that extracts heat from the sampled iron, and thereby influencing the solidification behaviour. Sand cups typically have open surfaces resulting in large radiation heat losses and thus imbalanced heat losses from the top, sides and bottom of the sample volume. In addition, the change in radiated heat transfer on solidification causes the thermal hot spot to move during the solidification process. Furthermore, sand cups, especially those filled from the open surface of the cup, are liable to variation in both the sample volume (operator consistency) and entrainment of oxygen during filling. Moreover, thermocouples found in conventional sand cups are rigidly mounted and consumed with each analysis. Therefore, variations in the thermocouple calibration and the location of the measurement junction directly influence the accuracy of the thermal analysis. The location of the simplified connection of these thermocouples lead to biases due to the uneven heat transfer conditions of the 2 wires as they pass separately from the sample to the rudimentary connector.

EP 1 925 936 discloses a sampling device for thermal analysis of molten metal which solves the drawbacks of conventional sampling devices constructed of chemically bonded sand. The sampling device is a container comprising one common filling inlet on the upper side of said container and at least two cavities, each cavity having a protective tube adapted for enclosing a temperature responsive member. The common filling inlet is branched into at least two filling channels ending in said cavities. The sampling device is manufactured of steel or a moulded fibrous refractory cloth material.

Other examples of previously known sampling devices are disclosed in WO 96/23206 and EP 1 034 419. Said sampling devices comprise a container intended to contain a sample quantity of liquid metal during analysis, and a sensor for thermal analysis. The container of such sampling devices is intended to be immersed in a molten bath of the material to be analysed in order to fill the container with a consistent sample quantity. The container is thereafter removed from the molten bath and the sample quantity is allowed to solidify while recording the time-dependent temperature change during said solidification.

While the sampling devices described above solve at least some of the problems associated with conventional sampling devices constructed of chemically bonded sand, they may be less preferred in certain circumstances. For example, they often tend to have higher production cost than sand cups. They are also not designed to fill multiple vessels simultaneously.

### Summary

The object of the invention is a sampling device for thermal analysis which may be easily manufactured in a cost-effective manner and which ensures that reliable results during thermal analysis are consistently achieved irrespective of the skill and discipline of the operator.

The object is achieved by a sampling device in accordance with independent claim 1. Embodiments are defined by the dependent claims.

The sampling device for thermal analysis of solidifying metal according to the present invention comprises an upper side and a lower side. The uppermost portion of the upper side defines a horizontal first plane. The sampling device comprises at least two cavities adapted to be filled with a molten metal to be analysed. The cavities are each intended to substantially enclose the molten metal during thermal analysis and are as such only provided with openings necessary for their function, such as the filling opening and a possible ventilation hole or channel for venting out gaseous substances from the cavity during filling. For the purpose of thermal analysis, a respective temperature responsive means is provided at least partly inside each of the cavities, and the cavities are thus adapted for containing such a temperature responsive means. The sampling device further comprises one common filling inlet having an inlet opening on the upper side of the sampling device. It is preferred that the common filling inlet is the single filling inlet of the sampling device. The common filling inlet is at a distance from the upper side of the sampling device branched into a plurality of filling channels, such as two filling channels. Each filling channel opens up into a respective cavity. The inlet opening of the common filling inlet is arranged in a second plane essentially parallel to the first plane below said first plane. Thus, the inlet opening of the common filling inlet is arranged vertically below the uppermost portion of the upper side of the sampling device.

Suitably the inlet opening of the common filling inlet may be arranged in a horizontal plane which is arranged at a distance from the first plane which is at least 20%, preferably at least 25%, of the distance between the first plane and a horizontal plane tangent to an uppermost internal surface of at least one of the first cavity and the second cavity. In case of cavities having different distances between the first plane and their respective uppermost internal surface, the horizontal plane tangent to an uppermost internal surface of a cavity is preferably the horizontal plane tangent to an uppermost internal surface of the cavity having the smallest distance to the first plane.

The inlet opening of the common filling inlet may be arranged essentially in a horizontal plane tangent to an uppermost internal surface of at least one of the first cavity and the second cavity.

The sampling device may furthermore comprise open ventilation channels or holes connecting a cavity through the upper surface to the open atmosphere. The purpose of such a ventilation hole or channel is to enable gaseous substances from the cavity to be vented out from the cavity during for example filling. Each cavity is suitably substantially enclosed except for the respective filling channel and the respective ventilation channel/hole.

The sampling device may further comprise a ring shaped recess arranged in the upper side of the sampling device radially outside of and concentric with the opening of the common filling inlet. The purpose of such a recess is to (at least temporarily) accommodate any excess molten metal after filling of the sampling device. Alternatively, the sampling device may comprise a run-off channel on the upper side of the device connected to the filling inlet. The purpose of such a channel is to drain away any excess molten metal from the filling inlet so that the total volume of the filling inlet is consistent from one sample to the next. The run-off channel is adapted to remove the excess molten in a safe direction, i.e. away from operators or other sensitive apparatus.

The cavities may or may not have the same internal volume, have the same geometrical shape, and/or be arranged with their respective centres in the same horizontal plane (i.e. with their centres arranged at the same vertical distance from the first plane). It is also plausible to arrange the cavities of the sampling device such that each of the cavities has its uppermost internal surface arranged in the same horizontal plane, irrespective of whether or not the cavities have the same internal volume and/or geometrical shape.

In the sampling device, a substance adapted to intentionally alter the solidification behaviour of the molten metal contained thereof during thermal analysis may be provided in at least one cavity. For example, if desired, the inner walls of one or more of the cavities facing the sample quantity may be provided with a reactive coating adapted to alter the solidification behaviour of the molten metal. Such a coating may for example be provided by brushing a slurry or the like of a suitable coating composition onto the inner wall of a cavity and allowing the coating composition to dry such as to form the coating. It is also possible to provide a coating on the walls of one cavity whereas another cavity of the sampling device is either free from such a coating or contains a different coating intended to alter the solidification in a different manner. Thereby, it may be possible to determine the solidification behaviour of the same melt simultaneously for different conditions or different purposes. Instead of a reactive coating, a powdery or granular substance may be provided to one or more cavities and only contained in the cavity without being coated on a wall of the cavity. The substance may also be contained in a sacrificial envelope to prevent its escape from the cavity during transport and handling. Alternatively, a paste of a substance may be adhered to a part of the wall of a cavity, for example as a "blob".

The sampling device may comprise additional cavities adapted to be filled with molten metal to be analysed if desired. For example, the sampling device may comprise three, four or five cavities. The cavities suitably share the same common filling inlet, and are suitably arranged at equal distances from the common filling inlet.

Moreover, the sampling device may suitably comprise a protective tube adapted to contain at least one temperature responsive means during thermal analysis. Such a protective tube may extend through at least one wall of the sampling device and be open in one end to enable inserting a temperature responsive means. The protective tube may for example extend radially through a cavity and comprise at least one end opening adapted for inserting a temperature responsive means into the protective tube. Alternatively, the protective tube may comprise a closed end and extend radially into a cavity such that the closed end is arranged essentially in the centre of said cavity. The protective tube may if desired comprise a plurality of temperature responsive means.

The present disclosure also relates to a kit for thermal analysis comprising at least two, preferably a plurality of, temperature responsive means, such as thermocouples, and a sampling device as disclosed above. The number of temperature responsive means generally correspond to the number of cavities in the sampling device, but could also be more than the number of cavities of the sampling device in order to obtain cooling curves from different locations within a cavity.

### Brief description of the drawings

- Fig. 1: illustrates a sand cup in accordance with prior art.
- Fig. 2: illustrates a cross sectional view of a sampling device in accordance with a first exemplifying embodiment.
- Fig. 3: illustrates a cross sectional view of a sampling device in accordance with a second exemplifying embodiment.
- Fig. 4: illustrates a cross sectional view of a sampling device in accordance with another exemplifying embodiment.
- Fig. 5: illustrates a cross sectional view of a sampling device in accordance with yet another exemplifying embodiment.
- Fig. 6: illustrates a cross sectional view of a sampling device in accordance with yet another exemplifying embodiment.
- Fig. 7: illustrates a cross sectional view of a sampling device in accordance with another exemplifying embodiment.
- Fig. 8: illustrates a cross sectional view of a sampling device in accordance with another exemplifying embodiment.
- Fig. 9: illustrates a cross sectional view of a sampling device in accordance with another exemplifying embodiment.
- Fig. 10: illustrates a cross sectional view of a sampling device in accordance with another exemplifying embodiment.
- Fig. 11: illustrates a top view of a sampling device in accordance with another exemplifying embodiment.
- Fig. 12: illustrates a top view of a sampling device in accordance with yet another exemplifying embodiment.

### Detailed description

The invention will be described below with reference to the accompanying drawings. The invention is not limited to the embodiments shown but may be varied within the scope of the appended claims. Moreover, the drawings shall not be considered drawn to scale as some features may be exaggerated in order to more clearly illustrate the features of the sampling device or the details thereof.

In the figures representing the present invention, the cavities are illustrated as sphere shaped which is a preferred embodiment. However, the cavities may also have other shapes such as oblate spheroid, sphere with a flat top or bottom, elipsoid, cube, square prism, regular polygon prism, 3 or more sided prism, etc. if desired without departing from the scope of the invention. Furthermore, the cavities may have the same shape, such as shown in the figures, or may be of different geometrical shapes if desired. Moreover, the size of the cavities may be the same or may differ from each other in the same sampling device to provide different solidification rates. In case of the cavities having different geometrical shape, the internal volume of the cavities need not necessarily be substantially different. The size and shape of the cavities can be adapted to the intended purpose by a person skilled in the art in view of the present disclosure.

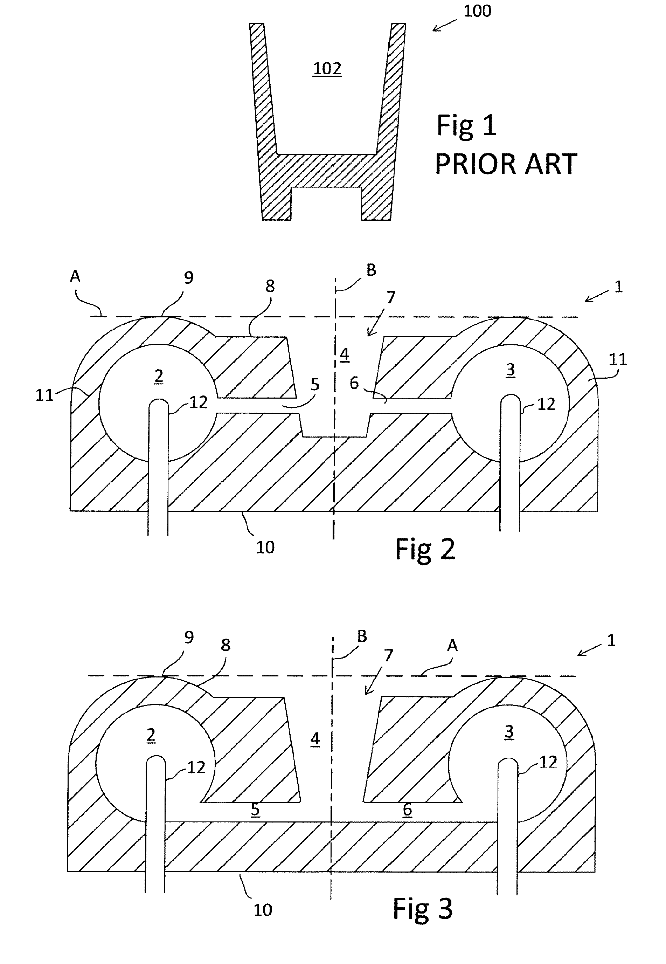

Figure 1 illustrates a so called sand cup 100 in accordance with prior art. The sand cup is constructed of chemically bonded sand and is open towards the surrounding atmosphere at the top of the container 102 adapted to comprise the molten metal to be analysed by thermal analysis. The open surface of the container 102 causes radiation heat losses from the surface of the molten metal which in turn influences the solidification of the molten metal. In essence, there is an imbalance of the heat loss which in turn does not enable sufficiently accurate readings in certain cases.

In contrast to the prior art shown with reference to Figure 1, the molten sample is essentially enclosed in the cavities of the sampling device according to the present invention. Thereby, the heat loss through radiation from an open surface of the molten metal is essentially eliminated. Essentially enclosed is in the present disclosure considered to mean that each one of the cavities is enclosed except for the desired openings in the walls of the respective cavity, said openings constituting the filling opening of the cavity, potential ventilation hole(s) and the opening(s) needed for inserting the temperature responsive means.

Figure 2 illustrates a cross sectional view of one exemplifying embodiment of the sampling device 1 in accordance with the present invention. The sampling device 1 comprises at least a first cavity 2 and at least one second cavity 3. The cavities 2, 3 are adapted to contain molten metal to be analysed by thermal analysis. Molten metal to be analysed is introduced into the respective cavities 2, 3 through a common filling inlet 4 which in turn is branched in to a plurality of filling channels 5, 6 inside the sampling device at a vertical distance from the inlet opening. The common filling inlet is preferably arranged essentially between the cavities such that the vertical central axis B of the filling inlet is arranged midway between the respective centres of the cavities. Furthermore, the common filling inlet 4 may suitably at least partly have the shape of a truncated cone in order to facilitate the filling of molten metal into the common filling inlet.

The common filling inlet 4 ensures that the molten metal is filled into the sampling device 1 through only one inlet opening 7, thereby ensuring that an operator need not ensure equal amounts to be filled into the cavities as would be the case if each cavity would be filled separately. Each filling channel 5, 6 opens into a respective cavity 2, 3 of the sampling device at an inlet opening of a cavity. As shown in Figure 2, a filling channel 5, 6 may open up into a cavity 2, 3 essentially at the same height as the centre of the cavity 2, 3.

Each of the cavities 2, 3 is provided with at least one temperature responsive means (not shown in the figure), at least during thermal analysis. The temperature responsive means is adapted to measure the temperature during the solidification of the molten metal and the measurements are recorded over time in order to determine the changes during the solidification process of the molten metal. In Figure 2, a protective tube 12 is illustrated in each of the cavities 2, 3. The protective tube 12 is intended to contain and protect a temperature responsive means during thermal analysis. It is however plausible to use expendable temperature responsive means, as in the current state of the art shown in Figure 1, which are consumed during the thermal analysis. The benefit of a possible protective tube 12 is that a temperature responsive means can be reused in another sampling device after the thermal analysis is completed, thus reducing the cost of the sampling device. Reusable temperature responsive means can be made to be more accurate than consumable counterparts without increasing cost. The sampling device 1 as such is only used for a single analysis and is not intended to be reused for another sample quantity.

As shown in Figure 2, the inlet opening 7 of the common filling inlet 4 is arranged on an upper surface or side 8 of the sampling device 1 but is arranged at a lower level than the uppermost surface 9 of the sampling device. This means that the inlet opening 7 of the common filling inlet 4 is arranged in a second plane parallel to a first horizontal plane A defined by (i.e. tangent to) an uppermost portion 9 of the upper side 8 of the sampling device 1. The second plane is thus arranged below the first plane A, i.e. closer to the bottom (lower side 10) of the sampling device. The fact that the inlet opening is arranged in said second plane ensures that the sampling device is compact and easy to handle. Furthermore, the fact the inlet opening is arranged in this manner ensures that the sampling device may be manufactured by the least amount of material possible, i.e. that the walls of the cavity can be made very thin, thereby minimising the risk of the walls 11 of the sampling device acting as a heat sink during thermal analysis. In case the walls of the sampling device would act as a heat sink, it could negatively influence the solidification of the molten sample thereby altering the accuracy of the results achieved during thermal analysis.

It has previously been considered necessary to arrange filling inlets well above the level of the cavities in order to ensure consistent filling of the cavities. However, the inventors have found that consistent filling of the cavities is still achieved despite the location of the filling inlet according to the present invention.

The sampling device according to the present invention comprises at least two cavities, irrespective of the embodiment thereof. This has the benefit of providing two separate results for the same molten metal sample, thereby increasing the accuracy of the readings. It is however also possible to analyse different aspects of the same melt at the same time by means of the two cavities. For example, one of the cavities may be free from any additions and thereby analysing the actual molten metal whereas another cavity may be provided with a substance influencing the solidification behaviour of the molten metal, for example with the purpose of studying the nucleating potential by adding an inoculant or analysing the carbon equivalent by adding sulphur and/or tellurium or by adding sulphur and/or reducible oxides to intentionally alter the growth behaviour of the graphite, or by adding materials to enhance the analysis of the solid state transformation of austenite to ferrite and pearlite. Addition of one or more substances adapted to alter the solidification behaviour of the molten metal may be made in accordance with any previously known method. For example, such a substance may be added in the form of a functional or reactive coating which is applied to the internal walls of a cavity by means of for example dipping, rinsing, spraying or brushing a coating composition comprising the substance. The substance may also in some cases be added in the form of a powder, granulate or the like merely introduced into the cavity without attaching the powder to the internal walls of the cavity, the powder/granulate optionally contained in a sacrificial envelope, or by forming a paste containing the substance, forming it into a small ball, "blob" or the like and adhering the ball to a part of the internal wall of the cavity.

Figure 3 illustrates a cross sectional view of another exemplifying embodiment of the sampling device 1 which corresponds to the embodiment shown in Figure 2 with the exception of the filling channels 5, 6 opening up into the cavities 2, 3 in a bottom portion of the respective cavities. It is also possible to allow the filling channels to open up into the cavities from the underside of the respective cavities such that the molten metal would be introduced essentially vertically upwards into the cavities.

The filling channels 5, 6 are illustrated in both Figure 2 and Figure 3 as essentially horizontal filling channels. It is however possible to arrange the filling channels slightly sloping upwards or downwards from the common filling inlet towards the cavities if desired and the present invention is not limited to horizontal filling channels.

While Figures 2 and 3 illustrate only two cavities, the sampling device may suitably comprise at least three cavities. In the same manner as shown in Figures 2 and 3, a sampling device comprising three or more cavities suitably has the common filling inlet arranged in the middle such that the central axis B thereof is arranged at essentially the same distance from the centres of each of the cavities. The use of three or more cavities allows for the evaluation of the solidification without any additives and also with different additives in the different cavities in a corresponding manner as previously disclosed with reference to a sampling device comprising two cavities.

It is preferred that the central axis B of the common filling inlet corresponds to the central axis of the sampling device for all of the embodiments disclosed herein.

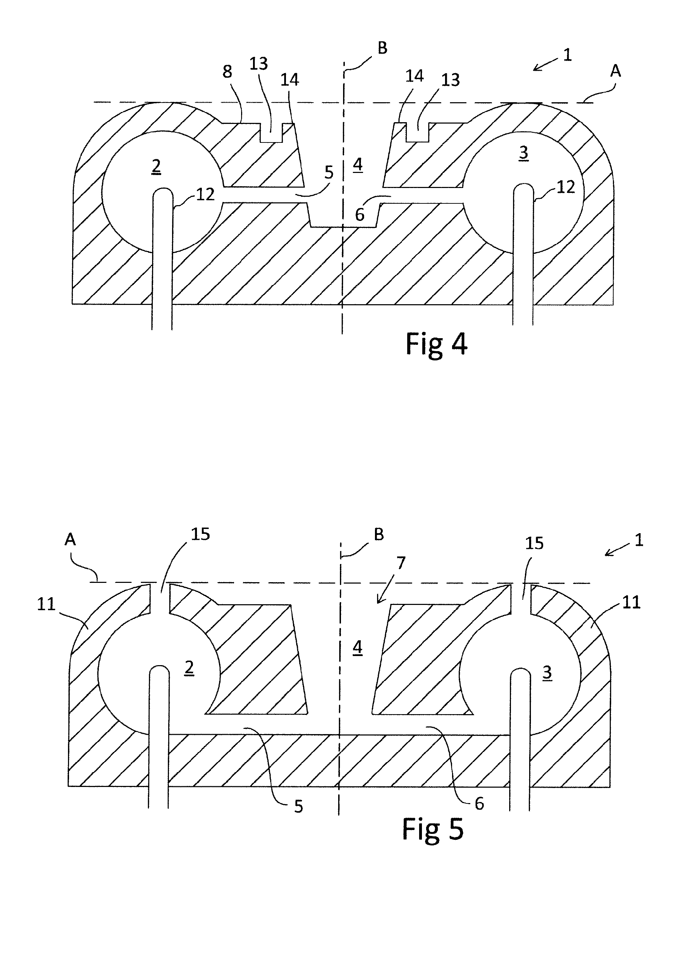

Figure 4 illustrates a cross sectional view of another exemplifying embodiment of the sampling device 1 similar to the one shown in Figure 2. However, as shown in Figure 4, the sampling device further comprises a ring shaped recess 13 in the upper side thereof. The recess is arranged radially outside of the common filling inlet 4 and concentric therewith. The purpose of the recess 13 is to accommodate any excess molten metal flowing over the edge 14 of the inlet opening of the common filling inlet when the cavities have been filled with molten metal. The recess 13 thus serves as trough for excess molten metal and provides a safety measure for avoiding molten metal to flow over on the sides of the sampling device.

The sampling device may suitably further comprise open ventilation holes or channels 15 in the walls of the cavities connecting each cavity 2, 3 with the open atmosphere above the sample, such as shown in the exemplifying embodiment shown in Figure 5. Such ventilation holes or channels serve the purpose of enabling air contained in the cavities before filling of the cavities, and depending on the material from which the sampling device is manufactured, volatile gasses liberated from the resin or possibly added substances when exposed to the molten metal, with molten metal to escape from the cavities during filling and prior to the onset of solidification, thereby ensuring that the cavities can be completely filled. Furthermore, the ventilation holes and channels may in certain cases additionally serve the purpose of at least partly allowing excess molten metal to escape from the cavities when the cavities become filled during filling. However, in view of the fact that the inlet opening 7 of the common filling inlet 4 is arranged at a lower level than the uppermost surface of the sampling device, such a rise of molten metal through the ventilation holes or channels 15 is likely to be minimal in most instances. These levels may also be adjusted to ensure that no molten metal is flushed through cavities where possibly added substances might be washed away via the ventilation holes or channels.

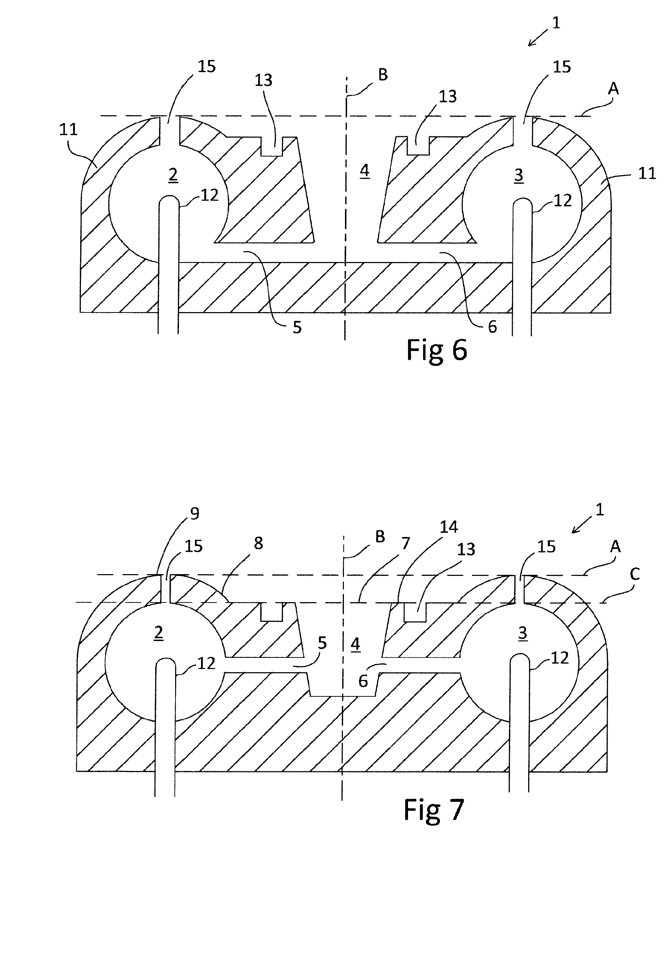

Figure 6 illustrates a cross sectional view of yet another exemplifying embodiment of the sampling device. In this case, the sampling device 1 comprises both the ring shaped recess 13 as shown in Figure 4 as well as the ventilation holes or channels 15 as shown in Figure 5. While the filling channels 5, 6 are opening up into the cavities in the bottom of the cavities, it is naturally plausible that the filling channels may open up into the cavities at any height, such as at a height corresponding to a centre of at least one of the cavities or at any height between such a height and the bottom portion of a cavity. It is also possible to arrange the filling channels such that they open up in the bottom of each cavity, thereby achieving that the filling of molten metal into the cavities is conducted essentially vertically upwards, i.e. the molten metal rises into the cavities.

In accordance with the present invention, the inlet opening of the common filling inlet is arranged in a second plane which is parallel to a first plane constituting a horizontal plane which is tangent to the uppermost part of the upper side of the sampling device. The second plane is arranged at lower height than the first plane and may be arranged at any height between the first plane and a plane tangent to an uppermost part of an internal surface of a cavity, excluding the first plane but including the plane tangent to an uppermost part of an internal surface of a cavity. In case the cavities have different size or are arranged at different vertical distances within the sampling device, the plane tangent to an uppermost part of an internal surface of a cavity corresponds to said plane of the cavity having such an uppermost part of an internal surface closest to the uppermost part of the sampling device (the first plane).

Figure 7 illustrates a cross sectional view of yet another exemplifying embodiment of the sampling device 1, similar to the exemplifying embodiment shown in Figure 6. The sampling device according to Figure 7 differs from the sampling device according to Figure 6 in the level of the filling channels 5, 6. Furthermore, the second plane C in which the inlet opening of the common filling inlet 4 is arranged is a plane tangent to the uppermost internal surface of a cavity. This means that the molten metal after filling will be at the same height in the cavities as in the common filling inlet after the filling has been completed and the cavities are completely filled.

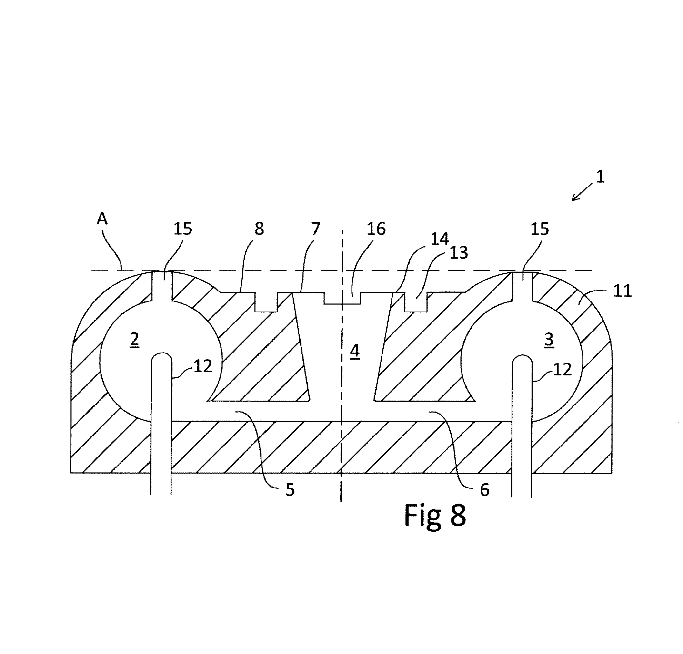

Figure 8 illustrates a cross sectional view of another exemplifying embodiment of the sampling device similar to the one shown in Figure 6. In contrast to the sampling device according to Figure 6, the sampling device according to Figure 8 also comprises at least one axially extending slot 16 in the upper portion of the common filling inlet, i.e. at the inlet opening 7 of the common filling inlet 4. The purpose of the slot is to allow excess molten metal during filling to flow through the slot 16 into the ring shaped recess 13 without needing to flow over the edge 14 of the inlet opening. The slot 16 thus provides a safety measure for limiting the risk of molten metal to flow on other portions of the sampling device.

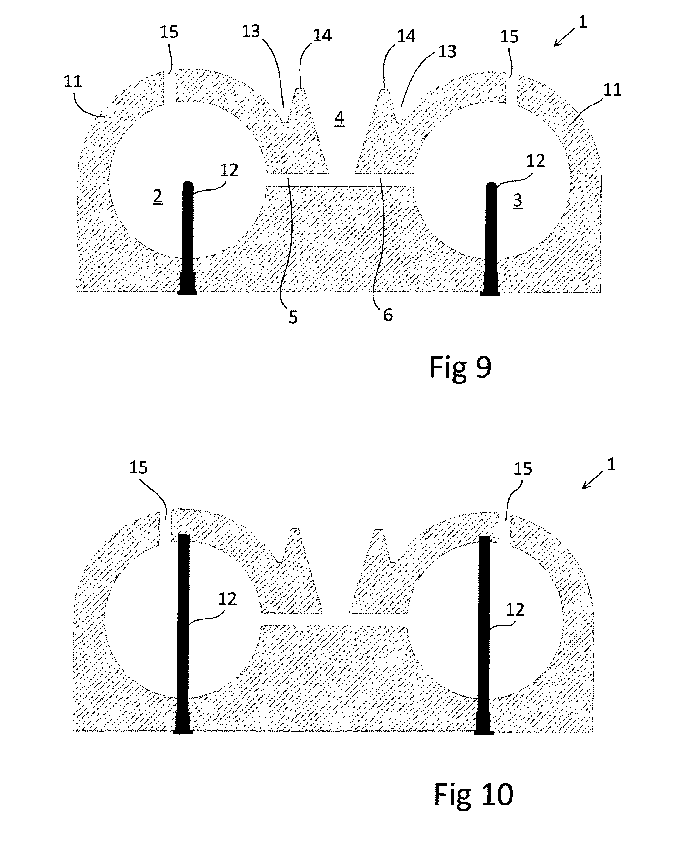

Figure 9 illustrates a cross sectional view of another exemplifying embodiment wherein the axial extension of the ventilation holes or channels 15 is arranged in a vertical plane parallel to a vertical plane through the centre of a cavity. In fact, the ventilation hole or channel 15 can be arranged anywhere in the wall 11 of the cavities. It is however preferred that the ventilation hole or channel is arranged in the upper portion of the cavities.

Arranging the ventilation hole or channel in a plane other than the vertical plane extending through the centre of a cavity, such as a plane parallel to said vertical plane, may also provide additional advantages. For example, it is then possible to allow a potential protective tube 12 to extend through the cavity and be supported in the wall on the opposite side of the cavity such as shown in Figure 10. By such an arrangement, the protective tube may be additionally secured in the cavity or may be produced as a tube with two open ends as opposed to one closed end. While not shown in Figure 10, it is also plausible to arrange the protective tube through the entire sampling device such that temperature responsive means may be introduced into the protective tube from any end thereof with the temperature measurement junction located at any preferred position within the cavity.

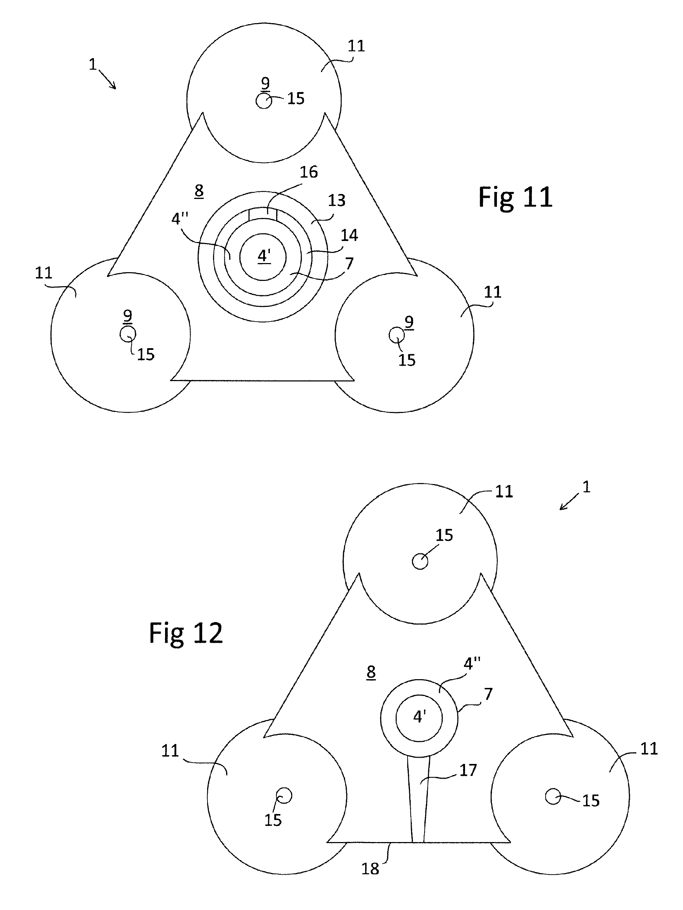

Figure 11 illustrates a top view of an exemplifying embodiment of a sampling device comprising three cavities each having a ventilation hole or channel 15. The common filling inlet is in the figure in the form of a truncated cone corresponding to the embodiments shown in the Figures 2-10, and the wall 4" of the cone as well as the bottom surface 4' are shown in the Figure 11. The inlet opening 7 of the common filling inlet 4 is arranged in a plane which is below the plane of the uppermost portions 9 of the upper side or surface 8 of the sampling device 1. The sampling device further comprises a ring shaped recess 13 and a axially extending slot 16 as previously disclosed with reference to Figure 8.

Figure 12 illustrates a top view of yet another exemplifying embodiment of a sampling device. A run-off channel 17, for example in the form of a groove or the like, is arranged in the upper side 8 of the sampling device running from the upper portion of the common filling inlet (illustrated in the figure by means of the axially extending wall 4" and the bottom surface 4') to a side surface 18 of the sampling device. The purpose of the run-off channel is to ensure that, when filling has been completed, any excess molten metal is removed from the sampling device in a desired and safe direction in order not to risk damaging any surrounding equipment possibly present in the vicinity of the sampling device or causing personal injury of an operator or other personnel. The run-off channel is preferably slightly sloping in the vertical direction from the common filling inlet towards the side surface of the sampling device to ensure that the excess molten metal readily flows towards the side surface. A receptacle, which may or may not be attached to the sampling device, may suitably be used to gather the excess molten metal at the downstream end of the run-off channel. The receptacle may possibly, if needed, be removed from the sampling device before thermal analysis.

While not shown in Figures 11 and 12, the sampling device may comprise both a ring shaped recess 13 as shown in Figure 11 and a run-off channel 17 as shown in Figure 12. In such a case, the upstream end of the run-off channel is preferably connected to the ring shaped recess such as to enable draining the excess molten metal from the ring-shaped recess.

The sampling device may be constructed of sand or sand based materials, especially chemically bonded sand. If desired the sampling device may in such a case also comprise an outer housing of another material, such as steel, if desired without departing from the scope of the invention. However, such an outer housing is not necessary and the sand itself provides sufficient stability of the sampling device. The sampling device may also be constructed of other types of materials such as synthetic sand or insulating refractories, a castable refractory, fibre cloth or coated steel. Also in the case of such materials, the sampling device may optionally comprise a housing (of the same or of a different material) if desired.

The sampling device may also optionally comprise external support means if desired. Such support means may for example be a supporting device serving the purpose of properly aligning temperature responsive means within one or more of the possible protective tubes during insertion thereof as well as during operation of the sampling device, i.e. during thermal analysis.

Any type of temperature responsive means that is suitable for measuring temperatures in molten metals, such as molten cast iron, may be used in connection with the sampling device according to the present invention. One example of such a temperature responsive means is a thermocouple.

The temperature responsive means may during thermal analysis suitably be arranged in a protective tube in order to ensure that the temperature responsive means is neither damaged nor consumed during the thermal analysis. Thus, the temperature responsive means may be removed from the sampling device after termination of the thermal analysis and reused in another sampling device. Furthermore, it is possible to arrange a plurality of temperature responsive means in the same protective tube if desired, for example for the purpose of obtaining cooling curves from different locations within a cavity, to increase the output of the temperature responsive means, or to reduce the measurement error.

The sampling device is not limited to the embodiments disclosed above and shown in the figures. For example, the protective tube need not be arranged vertically in the sampling device, but could for example be arranged essentially horizontally or angled to a horizontal and a vertical plane.

The sampling device according to the present invention is mainly developed for thermal analysis of grey cast iron (also known as lamellar cast iron -GJL), compacted graphite iron CGI (also known as vermicular graphite iron -GJV) and ductile iron (also known as nodular graphite iron or spheroidal graphite iron - GJS). However, the sampling device may also be used for analysing other molten substances, in particular molten metals, by means of thermal analysis.

## Claims

1. Sampling device (1) for thermal analysis of solidifying metal having an upper side (8) and a lower side (10), wherein the uppermost portion (9) of the upper side (8) defines a horizontal first plane (A), the sampling device comprising at least a first cavity (2) and a second cavity (3), the first and second cavities adapted to be filled with a molten metal to be analysed and each cavity adapted to comprise a temperature responsive means during thermal analysis, the sampling device further comprising one common filling inlet (4) having an inlet opening (7) on the upper side of the sampling device, the common filling inlet (4) branched into a plurality of filling channels (5, 6), each filling channel ending in one of the cavities (2, 3), **characterised in that** the inlet opening (7) of the common filling inlet (4) is arranged in a second plane essentially parallel to the first plane (A) and located below the first plane.

2. Sampling device according to claim 1, wherein the inlet opening (7) of the common filling inlet (4) is arranged essentially in a horizontal plane (C) which is tangent to an uppermost internal surface of at least one of the first cavity (2) and the second cavity (3).

3. Sampling device according to any one of the preceding claims, comprising open ventilation channels (15) connecting each of the cavities to the open atmosphere.

4. Sampling device according to claim 3, wherein each cavity (2, 3) is substantially enclosed except for the respective filling channel and the respective ventilation channel.

5. Sampling device according to any one of the preceding claims, further comprising a run-off channel (17) arranged in the upper side of the sampling device, said run-off channel adapted to guide possible excess molten metal from the common filling inlet in a predetermined direction towards a side surface (18) of the sampling device.

6. Sampling device according to any one of the preceding claims, further comprising a ring shaped recess (13) arranged in the upper side of the sampling device radially outside of and concentric with the opening (7) of the common filling inlet (4).

7. Sampling device according to claim 6, wherein a wall of the common filling inlet comprises an axially extending slot (16) in an upper portion thereof.

8. Sampling device according to any one of the preceding claims wherein the first and second cavities (2, 3) have the same internal volume.

9. Sampling device according to any one of the preceding claims wherein the second cavity (3) is provided with a substance adapted to intentionally alter the solidification behaviour of the molten metal contained therein during thermal analysis, and the first cavity (2) is free of such a substance.

10. Sampling device according to any one of the preceding claims wherein the sampling device comprises at least three cavities (2, 3) adapted to be filled with molten metal to be analysed.

11. Sampling device according to any one of the preceding claims wherein the centre of each of the cavities is arranged at the same distance from the first plane.

12. Sampling device according to any one of the preceding claims wherein each of the cavities comprises a protective tube (12) adapted to contain at least one temperature responsive means during thermal analysis, said protective tube preferably extending through at least one wall (11) of the sampling device.

13. Sampling device according to claim 12, wherein the protective tube (12) extends radially through a cavity and comprises at least one end opening adapted for inserting a temperature responsive means into the protective tube.

14. Sampling device according to claim 12, wherein the protective tube (12) comprises a closed end and extends radially into a cavity such that the closed end is arranged essentially in the centre of said cavity.

15. A kit of parts intended for thermal analysis of solidifying metal, said kit comprising at least two temperature responsive means and a sampling device (1) according to any one of the preceding claims.
